# EUROPÄISCHE PATENTSCHRIFT

(11) **EP 0 633 792 B1**
(45) Veröffentlichungstag und Bekanntmachung des Hinweises auf die Patenterteilung: **26.01.2000**
(21) Anmeldenummer: 93906520.7
(22) Anmeldetag: 13.03.1993
(51) Int. Cl.: A61M 15/00

(54) **SEPARATOR FÜR PULVERINHALATOREN**
SEPARATOR FOR POWDER INHALATORS
SEPARATEUR POUR INHALATEUR A POUDRE

(30) Priorität: 19.03.1992 DE 4208880
(43) Veröffentlichungstag der Anmeldung: 18.01.1995
(73) Patentinhaber: BOEHRINGER INGELHEIM INTERNATIONAL GmbH, 55218 Ingelheim am Rhein (DE); Boehringer Ingelheim Pharma KG, 55216 Ingelheim am Rhein (DE)
(72) Erfinder: GUPTE, Arun, Rajaram, D-6507 Ingelheim (DE); KLADDERS, Heinrich, D-6507 Ingelheim (DE)
(74) Vertreter: Laudien, Dieter, Dr.
(86) Internationale Anmeldenummer: EP9300582
(87) Internationale Veröffentlichungsnummer: WO9318811

(56) Entgegenhaltungen:
- EP-A- 0 069 715
- EP-A- 0 135 390
- EP-A- 0 237 507
- EP-B- 0 551 338
- WO-A-87/06475
- WO-A-92/05825
- US-A- 4 953 545

## Beschreibung

Die Erfindung betrifft einen Separator, der als Zusatz für Geräte zur Pulverinhalation bestimmt ist und der dazu dient, die nicht lungengängigen Partikeln in dem mit solchen Geräten erzeugten Aerosol von den inhalierbaren abzutrennen.

Ein derartiges Gerät ist schon aus der EP-B1-0 551 338 bekannt.

Bei der Inhalationstherapie von Atemwegserkrankungen ist es nötig, daß die pulverförmigen Arzneistoffe an den gewünschten Wirkungsort in der Lunge gelangen. Besonders wichtig ist dies bei den zunehmend inhalativ eingesetzten Corticosteroiden. Werden die Arzneistoffe im Mund-Rachenraum (oropharyngeal) abgeschieden, ist die Gefahr (insbesondere lokaler) Nebenwirkungen groß.

Die bekannten Pulverinhalatoren vermögen von dem Wirkstoff nur eine gewisse Menge so fein auszubringen ("inhalierbare Dosis"), daß er tief genug in die Lunge gelangt. Grobe Partikeln, wie die häufig auch durch Zusammenballung der mikronisierten Arzneipulver entstehen, werden dagegen oropharyngeal abgeschieden.

Die vorliegende Erfindung löst nun die Aufgabe, eine Vorrichtung zu schaffen, die bei der Inhalation von Aerosolen die gröberen Partikeln zerkleinert und/oder abtrennt und in der Hauptsache nur die lungengängigen Partikeln durchläßt. Diese Aufgabe wird mit den Merkmalen des Anspruchs 1 gelöst.

Das wesentliche Element eines solchen Separators ist eine Kammer mit Mitteln, durch die das Aerosol so geführt wird, daß die gröberen Pulveranteile abgeschieden und/oder zerkleinert werden, und die eine Einrichtung zum Anschluß des Inhalators sowie ein Mundstück aufweist.

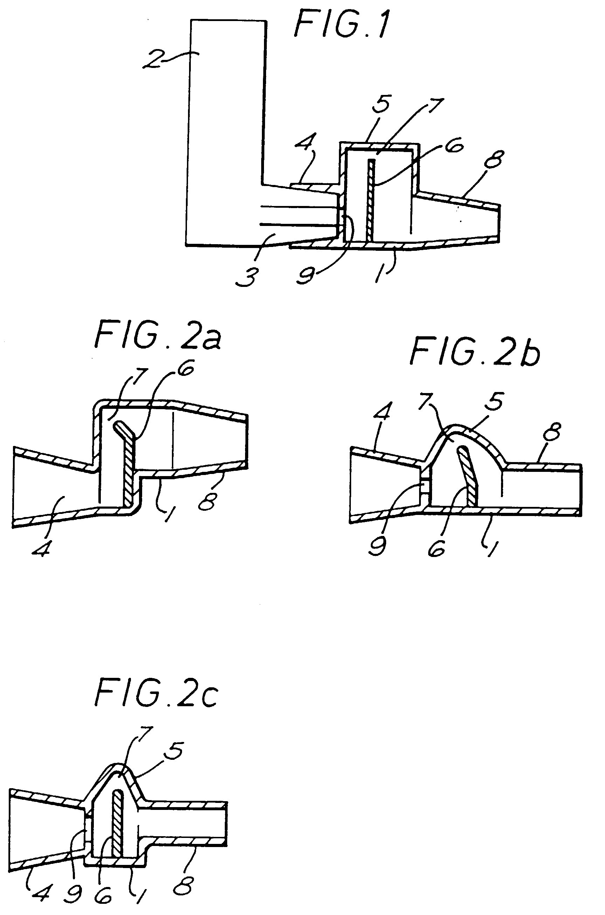

In den Figuren 1 und 2 sind erfindungsgemäß gestaltete Separatoren und Details davon schematisch dargestellt (in Benutzungsposition, soweit nichts anderes angegeben ist).

Figur 1 zeigt einen Längsschnitt durch einen solchen Separator mit dem damit verbundenen Inhalationsgerät üblicher Art.

Der Separator 1 ist auf das Inhalationsgerät 2 mit einem für die Aufnahme des Inhalatormundrohrs 3 vorgesehenen Stutzen 4 aufgesteckt. Das Aerosol wird gegen die in der Kammer 5 des Separators quer zur Strömungsrichtung angeordnete Prallplatte 6 geleitet und strömt an dieser entlang nach oben, so daß es (in der Anwendungsposition) oberhalb der Prallplatte 6 durch den Durchlaß 7 in den hinteren Teil der Kammer 5, weiter in das Mundstück 8 und von dort in den Mund bzw. die Atemwege des Patienten gelangt. Seitlich ist in dieser Ausführungsform zwischen der Wandung der Kammer und der Prallplatte kein Durchlaß. Die Prallplatte 6 kann hier wie auch in den anderen Versionen mit Mitteln zur Verbesserung der Trennwirkung versehen sein, z.B. mit Rippen gleicher oder unterschiedlicher Höhe quer zum Luftstrom, insbesondere auf der Prallseite.

Für eine ordnungsgemäße Funktion des Separators müssen die Abmessungen zum Teil aufeinander abgestimmt werden.

So soll der Abstand zwischen der Lufteinlaßöffnung 9 und der Prallplatte 6 etwa 2 bis 12 mm betragen, die Höhe der Prallplatte über dem höchsten Punkt der Lufteinlaßöffnung 9 etwa 10 bis 20 mm und die Höhe des Durchlasses 7 etwa 2 bis 10 mm und die Höhe des Durchlasses 7 etwa 2 bis 10 mm bei einer Breite von etwa 10 bis 30 mm.

Diese Zahlenangaben sind jedoch nur als Anhaltspunkte zu verstehen, da bei einer Änderung der Form gegebenenfalls auch Änderungen der Abmessungen zweckmäßig sind. Der Fachmann kann mit einfachen Versuchen die geeigneten Abmessungen ermitteln. Dabei ist für eine gute Separierung wichtig, daß der Luftstrom im Separator, insbesondere im Bereich vor der Prallplatte 6, nicht zu stark beschleunigt, sondern eher verlangsamt wird. Eine Beschleunigung kann jedoch bei solchen Aerosolen erwünscht sein, die größere Partikeln in Form relativ lockerer Agglomerate enthalten, welche bei ausreichend starkem Aufprall zerkleinert werden. Der Widerstand gegen die Strömung darf nicht zu groß sein; denn die Inhalationsgeräte sind für Patienten bestimmt, deren Lungenfunktion in vielen Fällen nicht die normale Leistung erreicht. Bei Strömungsquerschnitten von mehr als ca. 20 mm² ist dies in der Regel gewährleistet.

Die Kammer 5 des Separators kann verschiedene Formen haben; ihr Querschnitt - senkrecht zur Zeichnungsebene und parallel zu der Linie A-A' - kann beispielsweise rund, elliptisch, rechteckig oder polygonal, auch asymmetrisch sein. Auch der Schnitt in der Zeichnungsebene kann sehr verschieden geformt sein, etwa wie in den Figuren 2a bis 2c dargestellt. Der Durchlaß 7 kann nicht nur schlitzförmig, sondern z.B. rund, elliptisch, polygonal oder siebartig sein, wobei jedoch jeweils eine ausreichende Durchlässigkeit für den Luftstrom gewährleistet sein muß. Auch für die Prallwand 6 sind verschiedene Gestaltungen möglich; sie kann beispielsweise gebogen oder abgewinkelt sein, wie in Figur 2a oder 2c, ferner Unebeneheiten wie Noppen, regelmäßig angeordnete Prismen, Quader, Pyramiden oder Säulen mit beispielsweise polygonalem, rundem oder y-förmigem Querschnitt, Vertiefungen oder Querrillen bzw. Querrippen aufweisen.

Für praktische Zwecke ist es günstig, wenn der Separator in einzelne zusammensteck- bzw. -setzbare Teile zerlegt werden kann und/oder wenn die Kammer 5 sich durch einen Steck-, Klemm-, Klapp- oder Schraubmechanismus öffnen läßt, damit die abgeschiedenen gröberen Arzneimittelpartikeln von Zeit zu Zeit leichter entfernt werden können. Zwischen Inhalationsgerät und Separator sollte eine ausreichend fixierte, aber auch leicht lösbare Verbindung bestehen. Eine solche Verbindungsmöglichkeit kann in an sich bekannter Weise ausgeführt sein, sei es z.B. eine einfache Steckverbindung, bei der ein Wulst oder andere Vorsprünge in entsprechende Vertiefungen einrasten, eine Sperrklinke, eine Schraubverbindung oder ein Bajonettverschluß.

Der erfindungsgemäße Separator wird vorzugsweise aus Kunststoff hergestellt, kann jedoch auch ganz oder in Teilen aus Metall bestehen.

Der erfindungsgemäße Separator kann mit beliebigen Inhalationsgeräten kombiniert werden, bei deren Benutzung ein Aerosol mit einem störenden Anteil unerwünscht großer Partikeln entsteht. Demnach kann der erfindungsgemäße Separator beispielsweise mit Geräten nach DE-A 1566604, DE-A 3625685, DE-A 3927170, DE-A 4027390, DE-A 4027390, DE-A 4027391, EP-A 166294, EP-A 406893, GB 9026025, PCT/EP 91/01153, WO 90/13328 oder US 4889114 zusammen verwendet werden.

Beim Vergleich mit den Geräten ohne Separator zeigt sich, daß die Menge in inhalierfähigem Pulver im Aerosol (Teilchen bis zu 5,8 µm; "inhalierbare Dosis") durch den Separator nicht reduziert wird. Demgegenüber sinkt die Menge an größeren Teilchen auf weniger als ein Drittel.

Messungen dieser Art können in einem Humansimulator, d.h. einer Versuchsanordnung, bei welcher das Inhalationsgerät mit und ohne Separator in einen Luftstrom gebracht wird, der nach Menge und Strömungsgeschwindigkeit einem Atemzug angeglichen ist, durchgeführt werden.

Bei den erwähnten Versuchen wurde beispielsweise ein Luftstrom von 28 l/min angewendet, der jeweils für 1,5 sec durch den Inhalator geleitet wurde, während als Inhalationspulver Fenoterol mit einer mittleren Teilchengröße von 5,8 µm diente (jeweils 0,2 mg). Die inhalierbare Dosis wurde mit einem Kaskadenimpaktor der Fa. Andersen/USA bestimmt.

## Patentansprüche

1. Separator für Pulverinhalatoren, gekennzeichnet durch eine Kammer (5), die einen Stutzen (4) zur Aufnahme des Mundrohrs (3) eines Pulverinhalators sowie ein Mundstück (8) aufweist und in der ein Prall- und Leitmittel (6) im Strömungsweg des durch den Separator geleiteten Aerosols angebracht ist, wobei ein Durchlaß (7) zwischen dem Prall- und Leitmittel (6) und der Kammerwandung die Verbindung zum hinteren Teil der Kammer (5) und damit zum Mundstück (8) herstellt und wobei das Prall- und Leitmittel (6) mit seinem unteren Rand (in Benutzerhaltung) auf der Kammerwandung aufsitzt und in dem so gebildeten Winkel zwischen Prallmittel (6) und Kammerwandung abgetrennte Partikel zur Ablagerung gebracht werden.

2. Separator nach Anspruch 1, dadurch gekennzeichnet, daß der Durchlaß (7) schlitzförmig, rund, elliptisch oder polygonal ist.

3. Separator nach Anspruch 1 oder 2, dadurch gekennzeichnet, daß die Oberfläche der Prallplatte (6) uneben ist.

4. Separator nach Anspruch 1, 2 oder 3, dadurch gekennzeichnet, daß die Unebenheiten auf der Oberfläche der Prallplatte (6) kleine Vertiefungen, Erhöhungen oder Querrillen bzw. Querrippen sind.

5. Separator nach Anspruch 1, 2, 3 oder 4, dadurch gekennzeichnet, daß er aus zusammensteck- oder -schraubbaren oder klappbaren Teilen aufgebaut ist.

6. Separator nach Anspruch 1, 2, 3, 4 oder 5, in dem die Prallplatte (6) in ihrem oberen Teil gegen die Strömungsrichtung des Aerosols gebogen oder abgewinkelt ist.

## Claims

1. Separator for powder inhalers, characterised by a chamber (5) which has a connector (4) for receiving the mouth tube (3) of a powder inhaler, as well as a mouthpiece (8), and wherein an impact and deflection means (6) is arranged in the flow path of the aerosol passing through the separator, wherein an opening (7) between the impact and deflection means (6) and the chamber wall provides a connection to the rear part of the chamber (5) and hence to the mouthpiece (8) and wherein the impact and deflection means (6) rests with its lower edge (in the position of use) on the wall of the chamber, and any separated particles are deposited in the angle thus formed between the impact means (6) and the chamber wall.

2. Separator according to claim 1, characterised in that the opening (7) is slot-shaped, round, elliptical or polygonal.

3. Separator according to claim 1 or 2, characterised in that the surface of the impact plate (6) is uneven.

4. Separator according to claim 1, 2 or 3, characterised in that the unevennesses on the surface of the impact plate (6) are small depressions, elevations or transverse grooves or ribs.

5. Separator according to claim 1, 2, 3 or 4, characterised in that it is made up of parts which can be fitted or screwed together or folded.

6. Separator according to claim 1, 2, 3, 4 or 5, in which the impact plate (6), in the upper part thereof, is bent or angled counter to the direction of flow of the aerosol.

## Revendications

1. Séparateur pour inhalateurs à poudre, caractérisé par une chambre (5), qui comporte une tubulure (4) pour la réception du tube buccal (3) d'un inhalateur à poudre ainsi qu'un embout buccal (8) et dans laquelle un moyen de déviation et de guidage (6) est disposé dans le chemin d'écoulement de l'aérosol guidé dans le séparateur, où un passage (7) entre le moyen de déviation et de guidage (6) et la paroi de la chambre assure la liaison avec la partie postérieure de la chambre (5) et donc avec l'embout buccal (8) et où le moyen de déviation et de guidage (6) repose par son bord intérieur (dans la position d'utilisation) sur la paroi de la chambre, et les particules séparées sont amenées à sédimenter dans l'angle ainsi formé entre le moyen de déviation (6) et la paroi de la chambre.

2. Séparateur selon la revendication 1, caractérisé en ce que le passage (7) est en forme de fente, rond, elliptique ou polygonal.

3. Séparateur selon la revendication 1 ou 2, caractérisé en ce que la surface de la plaque de déviation (6) est inégale.

4. Séparateur selon la revendication 1, 2 ou 3, caractérisé en ce que les inégalités sur la surface de la plaque de déviation (6) sont de petites cavités, des élévations ou des rainures transversales ou des nervures transversales.

5. Séparateur selon la revendication 1, 2, 3 ou 4, caractérisé en ce qu'il est constitué par des parties qui peuvent être assemblées ou vissées ensemble ou rabattables.

6. Séparateur selon la revendication 1, 2, 3, 4 ou 5, dans lequel la plaque de déviation (6) est recourbée ou repliée dans sa partie supérieure à l'encontre du sens d'écoulement de l'aérosol.
